# EUROPEAN PATENT SPECIFICATION

(11) **EP 2 536 418 B1**
(45) Date of publication and mention of the grant of the patent: **09.01.2019**
(21) Application number: 11704913.0
(22) Date of filing: 18.02.2011
(51) Int. Cl.: A61K 8/44, A61K 8/49, A61Q 19/00, A61Q 19/02, A61Q 19/08, A61K 8/97, A61K 36/185, A61K 36/42

(54) **TOPICAL SKIN CARE FORMULATION**
TOPISCHE HAUTPFLEGEZUSAMMENSETZUNG
FORMULATION TOPIQUE DE SOIN DE LA PEAU

(30) Priority: 19.02.2010 US 306307 P
(43) Date of publication of application: 26.12.2012
(73) Proprietor: Mary Kay, Inc., Addison, TX 75001 (US)
(72) Inventor: HINES, Michelle, Addison TX 75001 (US)
(74) Representative: Uexküll & Stolberg
(86) International application number: PCT/US2011/025462
(87) International publication number: WO 2011/103449

(56) References cited:
- EP-A1- 1 949 889
- EP-A1- 2 092 934
- FR-A1- 2 849 776
- US-A1- 2004 042 996
- US-A1- 2008 038 388
- US-A1- 2010 247 563
- "International Cosmetic Ingredient Dictionary and Handbook", 2008, The Cosmetic, Toiletry, and Fragrance Association, XP002695104, vol. 1, pages 700-701, entries "CUCURBITA PEPO (PUMPKIN) FRUIT EXTRACT" and "CUCURBITA PEPO (PUMPKIN) SEED EXTRACT"
- "Mary Kay - TimeWise Body Targeted-Action Toning Lotion", , 2010, XP55058852, Retrieved from the Internet: URL:http://www.marykayintouch.com.sg/conte nt/images/PDF/Fact sheets/Timewise Body Toning Lotion Fact Sheet_ENG.pdf [retrieved on 2013-04-09]
- DATABASE GNPD [Online] MINTEL; May 2010 (2010-05), "Targeted-Action Toning Lotion", XP002695105, Database accession no. 1327082
- SCHAR M P: "ARGAN OL", EURO-COSMETICS, HEIDELBERG, DE, vol. 5, 1 January 1999 (1999-01-01), pages 45-47, XP001062004, ISSN: 0944-8942
- Erin P.: "Proline", , 21 July 2009 (2009-07-21), XP002695106, Retrieved from the Internet: URL:http://www.truthinaging.com/ingredient s/proline [retrieved on 2013-04-10]

## Description

### CROSS REFERENCE TO RELATED APPLICATIONS

This application claims the benefit of U.S. Provisional Application No. 61/306,307.

### BACKGROUND OF THE INVENTION

### A. Field of the Invention

The present invention relates generally to methods using topical skin care compositions.

### B. Description of Related Art

Ageing, chronic exposure to adverse environmental factors, malnutrition, fatigue, *etc.,* can change the visual appearance, physical properties, or physiological functions of skin in ways that are considered visually undesirable. Examples of such changes include reduced skin elasticity, reduced skin firmness, increased sagging of the skin, development of deep lines and wrinkles, development of pits or nodules in skin, *etc.* Current products on the market either do not provide a sufficient treatment option or require a multitude of different compositions to treat these physiological changes to skin.

### SUMMARY OF THE INVENTION

The present invention provides an effective solution to skin exhibiting reduced elasticity, reduced firmness, increased sagginess, dryness, flakiness, deep lines and wrinkles, pits or nodules, damage caused by ultraviolet radiation, *etc.* According to the present invention, the solution is topical application of a composition to skin in need thereof. The skin can be in the décolleté region (*e.g*., neck, shoulders, and/or upper chest), facial skin, and/or body skin (*e*.*g*., arms, hands, chest, abdomen, upper and lower back, legs, buttocks, feet, *etc*.). In certain aspects, the composition is not applied to facial skin. The composition includes a skin active ingredient selected from the group consisting of *argania spinosa* kernel extract, *cucurbita pepo* fruit extract, and an amino acid; and a dermatologically acceptable vehicle. The composition can include 0.0001, 0.001, 0.01, 0.1, 0.5, 1, 1.5, 2, 3, 4, 5, 6, 7, 8, 9, 10, 11, 12, 13, 14, 15, 16, 17, 18, 19, 20% or more by weight (w/w) of any one of or any combination of or all of these active ingredients. The composition includes a combination of *argania spinosa* kernel extract, *cucurbita pepo* fruit extract, and an amino acid. The composition can include 0.0001 to 2% or more by weight (w/w) of *argania spinosa* kernel extract, *cucurbita pepo* fruit extract, and an amino acid. The amino acid is a combination of proline and serine. The composition can be formulated as an emulsion, cream, or lotion. In particular aspects, the dermatologically acceptable vehicle can include water, an emollient, an alcohol, and a surfactant. The composition can include at least about 70% w/w water, 5% to 15% w/w of an emollient, 3 to 7% w/w of an alcohol, and 1 to 2% w/w of a surfactant. The emollient can include C12-15 alkyl benzoate, glycerin, propylene glycol, butylene glycol, or hydrogenated lecithin, or any combination thereof, or all of these ingredients. The alcohol can include stearyl alcohol, cetearyl alcohol, or cetyl alcohol, or any combination thereof, or all of these ingredients. The surfactant can be an alkanolamine surfactant (*e.g*., triethanolamine). The dermatologically acceptable vehicle can further include any one of, any combination of, or all of the following ingredients: dimethicone; ceteareth-20; carbomer; diazolidinyl urea; xanthan gum; lauramine oxide; disodium EDTA; fragrance; methylparaben; C9-15 alkyl phosphate; butylene glycol; propylparaben; sodium cocoyl glutamate; and/or phenoxyethanol. In particular embodiments, the dermatologically acceptable vehicle includes: water (q.s.); 6% w/w of C12-15 alkyl benzoate; 5.4425% w/w of glycerin; 1.9% w/w of stearyl alcohol; 1.463% w/w of cetearyl alcohol; 1.39% w/w of propylene glycol; 1.2% w/w of triethanolamine; 1.1% w/w of cetyl alcohol; 1% w/w of hydrogenated lecithin; 1% w/w of caffeine; 0.5% w/w of dimethicone; 0.437% w/w of ceteareth-20; 0.3655% w/w of carbomer; 0.3% w/w of diazolidinyl urea; 0.25% w/w of xanthan gum; 0.243% w/w of lauramine oxide; 0.225% w/w of disodium EDTA; 0.15% w/w of fragrance; 0.1301% w/w of methylparaben; 0.113% w/w of C9-15 alkyl phosphate; 0.10845% w/w of butylene glycol; 0.1% w/w of argania spinosa kernel extract; 0.1% w/w of crataegus oxyacantha fruit extract; 0.1% w/w of tocopheryl acetate; 0.055% w/w of cucurbita pepo seed or fruit extract; 0.03025% w/w of propylparaben; 0.03% w/w of sodium cocoyl glutamate; 0.02745% w/w of phenoxyethanol; 0.024% w/w of camellia sinensis leaf extract; 0.024% w/w of coffea arabica seed extract; 0.024% w/w of wintergreen extract; 0.0095% w/w of aesculus hippocastanum seed extract; 0.0095% w/w of serine; 0.0045% w/w of proline; 0.0045% w/w of centella asiatica extract; 0.00175% w/w of hydrolyzed myrtus communis leaf extract; and 0.001% w/w of euterpe oleracea fruit extract. The composition can also include a sunscreen/UV protecting agent (chemical or physical agents). The composition can be applied to skin located on a person's chest, arms, stomach, thighs, buttocks, face, *etc.* In particular aspects, the composition is applied to sagging skin, skin that has reduced elasticity, skin having deep lines or wrinkles, or skin pits or nodules. In certain aspects, any one of, any combination of, or all of the skin active ingredients, ingredients in the dermatologically acceptable vehicle, or any additional ingredients identified throughout this specification can be encapsulated for delivery to a target area such as skin. Non-limiting examples of encapsulation techniques include the use of liposomes, vesicles, and/or nanoparticles (*e*.*g*., biodegradable and non-biodegradable colloidal particles comprising polymeric materials in which the ingredient is trapped, encapsulated, and/or absorbed-examples include nanospheres and nanocapsules) that can be used as delivery vehicles to deliver such ingredients to skin. Such encapsulated ingredients can be incorporated into the compositions described throughout this specification.

Also disclosed is a method of increasing the integrity of the dermal-epidermal junction ("DEJ") comprising topically applying any one of the compositions disclosed throughout this specification to skin. This method can stimulate the production of proteins and enzymes in dermal and epidermal cells that aid in connecting the dermal layer to the epidermal layer. Not wishing to be bound by theory, it is believed that the combination of *argania spinosa* kernel extract and *cucurbita pepo* seed extract stimulate proteins that are vital to the health of the DEJ (*e.g.,* collagen and elastin). It is believed that pumpkin seed extract also limits the activity of particular enzymes that can cause collagen to breakdown or deteriorate (*e.g*., MMP-3). In addition to the combination of *argania spinosa* kernel extract and *cucurbita pepo* seed extract, amino acids such as proline and serine can also be included to provide further benefits to the DEJ. Therefore, the use of a combination of *argania spinosa* kernel extract and *cucurbita pepo* seed extract improves the health of the DEJ, and the combination of *argania spinosa* kernel extract, *cucurbita pepo* seed extract, proline, and serine can be used (*e.g*., proline and serine can aid in collagen formation and increase skin elasticity).

There is disclosed a method of stimulating dermal or epidermal cellular activity in sagging skin, skin that has reduced elasticity or reduced firmness, skin having deep lines and wrinkles, or skin pits or nodules comprising topically applying to the skin any one of the compositions disclosed throughout this specification. In particular aspects, the composition can include a combination of caffeine, *coffea arabica* seed extract, and *crataegus oxyacantha* fruit extract. The composition can include 0.001 to 2% w/w of any one, any combination of, or all of these ingredients.

Also disclosed is a method of reducing free-radical damage in skin or reducing oxidation of skin cells while also moisturizing skin. The skin can be skin over-exposed to UV radiation (*e.g*., sun burned skin), skin exposed to environmental pollutants (*e.g*., chemicals, smoke, *etc*.), aged skin (*e.g*., women 40 years of age and older), skin being treated with another composition (*e.g*., exfolliants) or method (*e.g*., laser surgery), *etc*.. The method includes comprising topically applying to skin in need thereof a composition comprising a combination of *euterpe oleracea* fruit extract, *hydrolyzed myrtus communis* leaf extract, *centella asiatica* extract, *camellia sinensis* leaf extract, *aesculus hippocastanum* seed extract, tocopheryl acetate, and glycerin. The composition can include 0.001% to 2% w/w of any one of, any combination of, or all of *euterpe oleracea* fruit extract, *hydrolyzed myrtus communis* leaf extract, *centella asiatica* extract, *camellia sinensis* leaf extract, *aesculus hippocastanum* seed extract, and/or tocopheryl acetate. The composition can also include 3 to 7% w/w of glycerin.

There is disclosed a composition and corresponding method of increasing the firmness and elasticity of skin, stimulating dermal or epidermal cellular activity in skin, reducing free-radical damage of skin, and moisturizing skin. The composition can include: (a) a combination of ingredients that firm skin comprising *argania spinosa* kernel extract, *cucurbita pepo* seed or fruit extract, and an amino acid; (b) a combination of ingredients that stimulate dermal or epidermal cellular activity comprising caffeine, *coffea arabica* seed extract, and *crataegus oxyacantha* fruit extract; (c) a combination of ingredients that reduce free-radical damage of skin comprising *euterpe oleracea* fruit extract, *hydrolyzed myrtus communis* leaf extract, *centella asiatica* extract, *camellia sinensis* leaf extract, and *aesculus hippocastanum* seed extract; (d) a combination of ingredients that moisturize skin comprising tocopheryl acetate and glycerin; and (e) a dermatologically acceptable vehicle. The method can include applying the composition to skin in need thereof. The composition can be formulated as an emulsion, cream, or lotion. In particular aspects, the dermatologically acceptable vehicle can include water, an emollient, an alcohol, and a surfactant. The composition can include at least about 70% w/w water, 5% to 15% w/w of an emollient, 3 to 7% w/w of an alcohol, and 1 to 2% w/w of a surfactant. The emollient can include C12-15 alkyl benzoate, glycerin, propylene glycol, butylene glycol, or hydrogenated lecithin, or any combination thereof, or all of these ingredients. The alcohol can include stearyl alcohol, cetearyl alcohol, or cetyl alcohol, or any combination thereof, or all of these ingredients. The surfactant can be an alkanolamine surfactant (*e.g*., triethanolamine).

The plant extract identified throughout this specification can be obtained from any part of the plant. Non-limiting examples include extracts obtain from the whole plant, leaves, stems, flowers, flower buds, bark, roots, fruit, seeds, and any mixture of such parts. By way of example, the extract can be obtained from the whole fruit (*e.g*., fruit pulp and seeds), the whole plant (*e.g*., the entire plant is used to produce the extract), a particular part of the plant at the exclusion of another part (*e.g*., seed extract isolated from other parts of the plant), *etc.* The extract can be water-based, alcohol-based, oil-based, gel-based *etc.*

In particular aspects, the compositions used in the method of the invention can be formulated as emulsions (*e.g*., oil-in-water, water-in-oil, silicone-in-water, water-in-silicone, water-in-oil-in-water, oil-in-water, oil-in-water-in-oil, oil-in-water-in-silicone, *etc*.), creams, lotions, solutions (*e.g*., aqueous or hydro-alcoholic solutions), anhydrous bases (*e.g*., lipstick or a powder), gels, ointments, milks, pastes, aerosols, solid forms, eye jellies, *etc..* The compositions can also be formulated for topical skin application at least 1, 2, 3, 4, 5, 6, 7, or more times a day during use. In other aspects of the present invention, compositions used in the method can be storage stable or color stable, or both. It is also contemplated that the viscosity of the composition can be selected to achieve a desired result, *e.g*., depending on the type of composition desired, the viscosity of such composition can be from about 1 cps to well over 1 million cps or any range or integer derivable therein (*e.g*., 2 cps, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000 cps, *etc.,* as measured on a Brookfield Viscometer using a TC spindle at 2.5 rpm at 25°C).

The compositions used in the method of the present invention can also be modified to have a desired oxygen radical absorbance capacity (ORAC) value. In certain non-limiting aspects, the compositions used in the method of the present invention or the plant extracts identified throughout this specification can be modified to have an ORAC value per mg of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 70, 80, 90, 95, 100, 200, 300, 400, 500, 600, 700, 800, 900, 1000, 2000, 3000, 4000, 5000, 6000, 7000, 8000, 9000, 10000, 15000, 20000, 30000, 50000, 100000 or more or any range derivable therein.

The compositions in non-limiting aspects can have a pH of about 6 to about 9. In other aspects, the pH can be 1, 2, 3, 4, 5, 6, 7, 8, 9, 10, 11, 12, 13, or 14.

Compositions used in the method of the present invention can have UVA and UVB absorption properties. The compositions can have an sun protection factor (SPF) of 2, 3, 4, 56, 7, 8, 9, 10, 11, 12, 13, 14, 15, 20, 25, 30, 35, 40, 45, 50, 55, 60, or more, or any integer or derivative therein. The compositions can be sunscreen lotions, sprays, or creams.

Kits that include the compositions used in the method of the present invention are also contemplated. In certain embodiments, the composition is comprised in a container. The container can be a bottle, dispenser, or package. The container can dispense a pre-determined amount of the composition. In certain aspects, the compositions is dispensed in a spray, dollop, or liquid. The container can include indicia on its surface. The indicia can be a word, an abbreviation, a picture, or a symbol.

Also contemplated is a product comprising a composition used in the method of the present invention. In non-limiting aspects, the product can be a cosmetic product. The cosmetic product can be those described in other sections of this specification or those known to a person of skill in the art. Non-limiting examples of products include a moisturizer, a cream, a lotion, a skin softener, a foundation, a night cream, a lipstick, a cleanser, a toner, a sunscreen, a mask, an anti-aging product, a deodorant, an antiperspirant, a perfume, a cologne, *etc.*

In one aspect, the compositions used in the method of the present invention do not include any one of any combination of or all of the following ingredients or characteristics: a ginsenoside compound, a ginseng flower extract, and/or niacinamide; *Argania spinosa* leaf extract, *Argania spinosa* fruit extract, and/or a triterpene fraction having erythrodiol, α-amyrine, β-amyrine, and lupeol; argan oil and/or an aloe extract; any one of the following plants or extracts thereof: *Eleutherococcus senticosus, Rhaponticum carthamoides, Panax ginseng, Panax quinquefolius, Pfaffia paniculada (Suma), Rhodiola rosea, Echinacea angustifolia, Echinacea purpurea, Ganoderma lucidum, Grifola frondosa, Hydrastis canadensis, Petiveria alliacea, Sutherlandia frutescens, Tabebuia avellanedae, Uncaria tomentosa, Angelica sinensis, Croton lechleri, Ginkgo biloba, Hydrocotyle asiatica, Ruscus aculeatus, and*/*or Vaccinium myrtillus*; washed oil bodies which are substantially intact and wherein the oil bodies are obtained from a plant; oil curd extracted from an oleaginous plant tissue, wherein the oil curd has an aqueous dispersion of oil bodies and a stability-enhancing amount of associated extrinsic protein material; a cold pressed botanical oil that has antioxidant properties; a pigment extracted from a botanical product that is adapted to stain skin; a broccoli sprout extract; an extract of *Lapsana communis*; a physical sunscreen agent such as titanium dioxide and/or zinc oxide and/or a chemical sunscreen agent (*e.g*., avobenzone, octocrylene, oxybenzone, *etc*.); an extract of tender coconut water and/or a fruit ring extract of *Garcinia cambogia*; an ingredient to stimulate human beta defensins of type 2 and/or type 3; *Sambucus nigra* and/or *Echinacea purpurea*; a glycoside extract of asiatic acid; an effective amount of ingredients that reduce glycation in skin tissue; rosemary extract; and/or cyclomethicone.

It is contemplated that any embodiment discussed in this specification can be implemented with respect to any method of the invention.

In one embodiment, the topical skin compositions of the current invention are pharmaceutically elegant. "Pharmaceutically elegant" describes a composition that has particular tactile properties which feel pleasant on the skin (*e.g*., compositions that are not too watery or greasy, compositions that have a silky texture, compositions that are non-tacky or sticky, *etc*.). Pharmaceutically elegant can also relate to the creaminess or lubricity properties of the composition or to the moisture retaining properties of the composition.

"Keratinous tissue" includes keratin-containing layers disposed as the outermost protective covering of mammals and includes, but is not limited to, skin, hair and nails.

"Topical application" means to apply or spread a composition onto the surface of keratinous tissue. "Topical skin composition" includes compositions suitable for topical application on keratinous tissue. Such compositions are typically dermatologically-acceptable in that they do not have undue toxicity, incompatibility, instability, allergic response, and the like, when applied to skin. Topical skin care compositions of the present invention can have a selected viscosity to avoid significant dripping or pooling after application to skin.

The term "about" or "approximately" are defined as being close to as understood by one of ordinary skill in the art, and in one non-limiting embodiment the terms are defined to be within 10%, preferably within 5%, more preferably within 1%, and most preferably within 0.5%.

The terms "inhibiting" or "reducing" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions used in the method of the present invention can comprise, consist essentially of, or consist of the claimed ingredients. In one aspect, compositions consisting essentially of the claimed ingredients excludes ingredients that would materially affect a given composition's ability to firm skin, increase the elasticity of skin, stimulate dermal or epidermal cellular activity of skin to increase the connection between the dermal and epidermal layers, reduce or prevent free-radical damage or oxidative damage of skin, moisturize skin, and/or reduce or prevent dry skin or flaky skin.

### DESCRIPTION OF ILLUSTRATIVE EMBODIMENTS

In today's image conscious society, people are continually looking for a product that can improve the visual appearance of their skin. Oftentimes, ageing, chronic exposure to adverse environmental factors, malnutrition, fatigue, *etc*., can change the visual appearance, physical properties, or physiological functions of skin in ways that are considered visually undesirable.

For instance, a healthy dermal-epidermal junction (DEJ), where the dermal layer meets the epidermal layer, can result in skin that appears lifted and toned. The DEJ includes: (1) a basal cell plasma membrane having hemidesmosomes (which are present on keratinocytes and attach the cell to the extracellular matrix); (2) the lamina lucida; (3) the basal lamina; and (4) the sub-basal lamina fibrous components, including anchoring fibrils, dermal microfibril bundles, and collagen fibers. Additional cells such as melanocytes and Merkel cells can also be present in the DEJ. The DEJ functions include: (1) epidermal-dermal adherence, (2) mechanical support for the epidermis, and (3) a barrier to the exchange of cells and of some large molecules across the junction. The DEJ can weaken due to the natural aging process and external factors. This can result in reduced skin elasticity, reduced skin firmness, increased sagging of the skin, development of deep lines and wrinkles, development of pits and/or nodules in skin, *etc.*

The inventor discovered that a unique combination of skin active ingredients can maintain or even improve the health of the DEJ. When this combination of ingredients is placed in a dermatologically acceptable vehicle, the end result is a topical skin care composition that can treat or prevent a wide range of skin conditions ranging from reduced elasticity, reduced firmness, increased sagginess, dryness, flakiness, deep lines and wrinkles, pits or nodules, damage caused by ultraviolet radiation, *etc.* This can be achieved in a single topical skin care composition used in the method of the invention.

These and other non-limiting aspects of the present invention are described in further detail below.

### A. Skin Active Ingredients

Skin active ingredients that can be used in the compositions used in the method of the present invention include (but are not limited to) caffeine, *argania spinosa* kernel (native to the Mediterranean) extract, *crataegus oxyacantha* fruit (native to Western Europe) extract, tocopheryl acetate, *cucurbita pepo* (native to regions all over the world) seed or fruit extract, *camellia sinensis* (native to China) leaf extract, *coffea arabica* (native to Yemen) seed extract, wintergreen extract, *aesculus hippocastanum* (native in Southeast Europe, Greece, Albania, Republic of Macedonia, Serbia, and Bulgaria) seed extract, an amino acid, *centella asiatica* (native to India, Sri Lanka, northern Australia, Indonesia, Iran, Malaysia, Melanesia, Papua New Guinea, and other parts of Asia) extract, *hydrolyzed myrtus communis* (native to Europe and North Africa) leaf extract, and/or *euterpe oleracea* (native to Brazil) fruit extract. Descriptions of each of these ingredients can be found in the International Cosmetic Ingredient Dictionary and Handbook, 12th Edition (2008) ("CTFA Handbook"), the relevant pages of which are incorporated by reference. Further, this reference also provides a list of companies that supply these ingredients. The following commercially available extracts can be used: (1) for *hydrolyzed myrtus communis* leaf extract, Longevicell C provided by Silab (Cedex, FRANCE); (2) for *argania spinosa* kernel extract, Argatensyl provided by Laboratories Serobiologiques (Paris, FRANCE); (3) for *crataegus oxyacantha* fruit extract, Hawthorne Berry Extract provided by Naturex/Pure World Botanicals (South Hackensack, New Jersey, USA); (4) for *cucurbita pepo* seed extract, Pumpkin Seed Extract provided by Draco Natural Products (San Jose, California, USA); (5) for *euterpe oleracea* fruit extract, Acai Fruit Extract provided by Carrubba Inc. (Milford, Connecticut, USA); and (6) for a combination of camellia sinensis leaf extract, coffea arabica seed extract, centella asiatica extract, and aesculus hippocastanum seed extract, Slimming Phytoamine Biocomplex provided by Alban Muller International (Vincennes, FRANCE). These listed commercially available extracts were used to obtain the data in Example 2 of this application.

For the plant extracts, the inventor also contemplates that any part of the plant can be used to make the extract. Non-limiting examples include extracts obtain from the whole plant, leaves, stems, flowers, flower buds, bark, roots, fruit, seeds, and any mixture of such parts. By way of example, the extract can be obtained from the whole fruit (*e.g*., fruit pulp and seeds), the whole plant (*e.g*., the entire plant is used to produce the extract), a particular part of the plant at the exclusion of another part (*e.g*., seed extract isolated from other parts of the plant), *etc.* The extract can be water-based, alcohol-based, oil-based, glycol/alcoholic based, gel-based *etc.*

In addition to purchasing the plant extracts, a person of ordinary skill can also be able to isolate them by using any suitable method known in the art. In one non-limiting example, the whole plant (or any part of the plant-*e*.*g*., leaves, stems, bark, roots, fruit, flowers or flower buds, fruit, seeds, seed pods, *etc*.) can be disrupted by mechanical means which results in a puree. The puree can then be processed to be substantially free of impurities or undesired solids. The puree can then be poured into a shallow vessel and quickly exposed to low temperature, *i.e*., flash frozen, for example at -20° C. or lower, under a vacuum for removal of water content (lyophilization). The resultant extract can then be used in the compositions of the present invention.

The combination of *argania spinosa* kernel extract, *cucurbita pepo* seed or fruit extract, and an amino acid can be used to firm skin or increase the elasticity of skin. The combination of caffeine, *coffea arabica* seed extract, and *crataegus oxyacantha* fruit extract can be used to stimulate dermal or epidermal cellular activity. The combination of *euterpe oleracea* fruit extract, *hydrolyzed myrtus communis* leaf extract, *centella asiatica* extract, *camellia sinensis* leaf extract, and *aesculus hippocastanum* seed extract can be used to reduce free-radical damage or oxidation of skin. The combination of tocopheryl acetate and glycerin can be used to moisturize skin for prolonged periods of time (up to 24 hours based on a single application).

The inventor also discovered a way to incorporate each of these actives into a single composition through a dermatologically acceptable vehicle. The end result is a multi-beneficial composition for skin.

### B. Dermatologically Acceptable Vehicle

A dermatologically acceptable vehicle or carrier can be used to deliver the skin actives to skin. In one non-limiting aspect, such a vehicle or carrier can include water, an emollient, an alcohol, and a surfactant. Non limiting examples of emollients include C12-15 alkyl benzoate, glycerin, propylene glycol, butylene glycol, and/or hydrogenated lecithin. Non-limiting examples of alcohol include stearyl alcohol, cetearyl alcohol, and/or cetyl alcohol. Non-limiting examples of surfactants include alkanolamine based surfactants (*e.g*., triethanolamine).

The dermatologically acceptable vehicle can be made by simple mixing of the ingredients, while using an appropriate amount of heating to obtain a homogenous mixture. A non-limiting example of such a vehicle is disclosed in the Examples of this specification. Additionally, a person having ordinary skill in the formulations art can use appropriate methods for making a dermatologically acceptable vehicle having the ingredients identified throughout the specification.

### C. Compositions of the Present Invention

### 1. Combinations and Amounts of Ingredients

It is contemplated that the compositions used in the method of the present invention can include any one of the skin active ingredients, ingredients in the dermatologically acceptable vehicle, or any combination of these ingredients. The compositions can also include additional ingredients described throughout this specification. The concentrations of the skin actives, ingredients in the dermatologically acceptable vehicle, or any additional ingredients can vary. In non-limiting embodiments, for example, the compositions can include in their final form, for example, at least about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99% or more, or any range or integer derivable therein, of at least one of, any combination of, or all of the skin actives, ingredients in the dermatologically acceptable vehicle, or any additional ingredients disclosed in this specification. In non-limiting aspects, the percentage of such ingredients can be calculated by weight or volume of the total weight of the compositions. The concentrations can vary depending on the desired effect of the compositions or on the product into which the compositions are incorporated.

### 2. Composition Vehicles

The compositions used in the method of the present invention can be formulated into all types of vehicles. Non-limiting examples of suitable vehicles include emulsions (*e.g*., oil-in-water, water-in-oil, silicone-in-water, water-in-silicone, water-in-oil-in-water, oil-in-water, oil-in-water-in-oil, oil-in-water-in-silicone, *etc*.), creams, lotions, solutions (both aqueous and hydro-alcoholic), anhydrous bases (such as lipsticks and powders), gels, ointments, pastes, milks, liquids, aerosols, solid forms, or eye jellies. Variations and other appropriate vehicles will be apparent to the skilled artisan and are appropriate for use in the present invention. In certain aspects, the concentrations and combinations of the ingredients can be selected in such a way that the combinations are chemically compatible and do not form complexes which precipitate from the finished product.

It is also contemplated that the skin actives, ingredients in the dermatologically acceptable vehicle, or any additional ingredients identified throughout this specification can be encapsulated for delivery to a target area such as skin. Non-limiting examples of encapsulation techniques include the use of liposomes, vesicles, and/or nanoparticles (*e*.*g*., biodegradable and non-biodegradable colloidal particles comprising polymeric materials in which the ingredient is trapped, encapsulated, and/or absorbed-examples include nanospheres and nanocapsules) that can be used as delivery vehicles to deliver such ingredients to skin (*see, e.g*., U.S. Patent 6,387,398; U.S. Patent 6,203,802; U.S. Patent 5,411,744; Kreuter 1988).

### 3. Products

The compositions used in the method of the present invention can be incorporated into cosmetic products, food-based products (*e.g*., fortified water, energy drinks, nutritional drinks, vitamins, supplements, solid foods), pharmaceutical products, *etc.* Non-limiting examples of cosmetic products include sunscreen products, sunless skin tanning products, hair products (*e.g*., shampoos, conditioners, colorants, dyes, bleaches, straighteners, and permanent wave products), fingernail products, moisturizing creams, skin creams and lotions, softeners, day lotions, gels, ointments, foundations, night creams, lipsticks and lip balms, cleansers, toners, masks, deodorants, antiperspirants, exfoliating compositions, shaving-related products (*e.g*., creams, "bracers" and aftershaves), pre-moistened wipes and washcloths, tanning lotions, bath products such as oils, foot care products such as powders and sprays, skin colorant and make-up products such as foundations, blushes, rouges eye shadows and lines, lip colors and mascaras, baby products (*e.g*., baby lotions, oils, shampoos, powders and wet wipes), and skin or facial peel products. Additionally, the cosmetic products can be formulated as leave-on or rinse-off products.

### 4. Additional Ingredients

Compositions used in the method of the present invention can include additional ingredients. Non-limiting examples of additional ingredients include cosmetic ingredients (both active and non-active) and pharmaceutical ingredients (both active and non-active).

### a. Cosmetic Ingredients

The CTFA Handbook describes a wide variety of non-limiting cosmetic ingredients that can be used in the context of the present invention. Examples of these ingredient classes include: fragrances (artificial and natural), dyes and color ingredients (*e.g*., Blue 1, Blue 1 Lake, Red 40, titanium dioxide, D&C blue no. 4, D&C green no. 5, D&C orange no. 4, D&C red no. 17, D&C red no. 33, D&C violet no. 2, D&C yellow no. 10, and D&C yellow no. 11), adsorbents, emulsifiers, stabilizers, lubricants, solvents, moisturizers (including, *e.g*., emollients, humectants, film formers, occlusive agents, and agents that affect the natural moisturization mechanisms of the skin), water-repellants, UV absorbers (physical and chemical absorbers such as paraaminobenzoic acid ("PABA") and corresponding PABA derivatives, titanium dioxide, zinc oxide, *etc*.), essential oils, vitamins (*e.g*., A, B, C, D, E, and K), trace metals (*e.g*., zinc, calcium and selenium), anti-irritants (*e.g*., steroids and non-steroidal anti-inflammatories), botanical extracts (*e.g*., aloe vera, chamomile, cucumber extract, ginkgo biloba, ginseng, and rosemary), anti-microbial agents, antioxidants (*e.g*., BHT and tocopherol), chelating agents (*e.g*., disodium EDTA and tetrasodium EDTA), preservatives (*e.g*., methylparaben and propylparaben), pH adjusters (*e.g*., sodium hydroxide and citric acid), absorbents (*e.g*., aluminum starch octenylsuccinate, kaolin, corn starch, oat starch, cyclodextrin, talc, and zeolite), skin bleaching and lightening agents (*e.g*., hydroquinone and niacinamide lactate), humectants (*e.g*., glycerin, propylene glycol, butylene glycol, pentylene glycol, sorbitol, urea, and manitol), exfoliants (*e.g*., alpha-hydroxyacids, and beta-hydroxyacids such as lactic acid, glycolic acid, and salicylic acid; and salts thereof) waterproofing agents (*e.g*., magnesium/aluminum hydroxide stearate), skin conditioning agents (*e.g*., aloe extracts, allantoin, bisabolol, ceramides, dimethicone, hyaluronic acid, and dipotassium glycyrrhizate), thickening agents (*e.g*., substances which that can increase the viscosity of a composition such as carboxylic acid polymers, crosslinked polyacrylate polymers, polyacrylamide polymers, polysaccharides, and gums), and silicone containing compounds (*e.g*., silicone oils and polyorganosiloxanes). The following provides specific non-limiting examples of some of the additional ingredients that can be used with the compositions of the present invention.

### i. Sunscreen Agents

UV absorption agents that can be used in combination with the compositions of the present invention include chemical and physical sunblocks. Non-limiting examples of chemical sunblocks that can be used include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, *etc*.), anthranilates, ethyl urocanate, homosalate, octisalate, dibenzoylmethane derivatives (*e.g*., avobenzone), octocrylene, octyl triazone, digalloy trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutyiphenol, and bis-ethylhexyloxyphenol methoxyphenyltriazine, 4-methylbenzylidenecamphor, and isopentyl 4-methoxycinnamate. Non-limiting examples of physical sunblocks include, kaolin, talc, petrolatum and metal oxides (*e.g*., titanium dioxide and zinc oxide).Compositions of the present invention can have UVA and UVB absorption properties. The compositions can have an sun protection factor (SPF) of 2, 3, 4, 56, 7, 8, 9, 10, 11, 12, 13, 14, 15, 20, 25, 30, 35, 40, 45, 50, 55, 60, 70, 80, 90 or more, or any integer or derivative therein.

### ii. Moisturizing Agents

Non-limiting examples of moisturizing agents that can be used with the compositions used in the method of the present invention include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrollidone carboxylic acid, potassium PCA, propylene glycol, sodium glucuronate, sodium PCA, sorbitol, sucrose, trehalose, urea, and xylitol.

Other examples include acetylated lanolin, acetylated lanolin alcohol, acrylates/C10-30 alkyl acrylate crosspolymer, acrylates copolymer, alanine, algae extract, aloe barbadensis, aloe-barbadensis extract, aloe barbadensis gel, althea officinalis extract, aluminum starch octenylsuccinate, aluminum stearate, apricot (prunus armeniaca) kernel oil, arginine, arginine aspartate, arnica montana extract, ascorbic acid, ascorbyl palmitate, aspartic acid, avocado (persea gratissima) oil, barium sulfate, barrier sphingolipids, butyl alcohol, beeswax, behenyl alcohol, beta-sitosterol, BHT, birch (betula alba) bark extract, borage (borago officinalis) extract, 2-bromo-2-nitropropane-1,3-diol, butcherbroom (ruscus aculeatus) extract, butylene glycol, calendula officinalis extract, calendula officinalis oil, candelilla (euphorbia cerifera) wax, canola oil, caprylic/capric triglyceride, cardamon (elettaria cardamomum) oil, carnauba (copernicia cerifera) wax, carrageenan (chondrus crispus), carrot (daucus carota sativa) oil, castor (ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (anthemis nobilis) oil, cholesterol, cholesterol esters, cholesteryl hydroxystearate, citric acid, clary (salvia sclarea) oil, cocoa (theobroma cacao) butter, coco-caprylate/caprate, coconut (cocos nucifera) oil, collagen, collagen amino acids, corn (zea mays)oil, fatty acids, decyl oleate, dextrin, diazolidinyl urea, dimethicone copolyol, dimethiconol, dioctyl adipate, dioctyl succinate, dipentaerythrityl hexacaprylate/hexacaprate, DMDM hydantoin, DNA, erythritol, ethoxydiglycol, ethyl linoleate, eucalyptus globulus oil, evening primrose (oenothera biennis) oil, fatty acids, tructose, gelatin, geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (vitis vinifera) seed oil, hazel (corylus americana) nut oil, hazel (corylus avellana) nut oil, hexylene glycol, honey, hyaluronic acid, hybrid safflower (carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, imidazolidinyl urea, iodopropynyl butylcarbamate, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (jasminum officinale) oil, jojoba (buxus chinensis) oil, kelp, kukui (aleurites moluccana) nut oil, lactamide MEA, laneth-16, laneth-10 acetate, lanolin, lanolin acid, lanolin alcohol, lanolin oil, lanolin wax, lavender (lavandula angustifolia) oil, lecithin, lemon (citrus medica limonum) oil, linoleic acid, linolenic acid, macadamia ternifolia nut oil, magnesium stearate, magnesium sulfate, maltitol, matricaria (chamomilla recutita) oil, methyl glucose sesquistearate, methylsilanol PCA, microcrystalline wax, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (olea europaea) oil, orange (citrus aurantium dulcis) oil, palm (elaeis guineensis) oil, palmitic acid, pantethine, panthenol, panthenyl ethyl ether, paraffin, PCA, peach (prunus persica) kernel oil, peanut (arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG40 sorbitan peroleate, PEG-5 soy sterol, PEG-10 soy sterol, PEG-2 stearate, PEG-8 stearate, PEG-20 stearate, PEG-32 stearate, PEG40 stearate, PEG-50 stearate, PEG-100 stearate, PEG-150 stearate, pentadecalactone, peppermint (mentha piperita) oil, petrolatum, phospholipids, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, potassium sorbate, potassium stearate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, quaternium-15, quaternium-18 hectorite, quaternium-22, retinol, retinyl palmitate, rice (oryza sativa) bran oil, RNA, rosemary (rosmarinus officinalis) oil, rose oil, safflower (carthamus tinctorius) oil, sage (salvia officinalis) oil, salicylic acid, sandalwood (santalum album) oil, serine, serum protein, sesame (sesamum indicum) oil, shea butter (butyrospermum parkii), silk powder, sodium chondroitin sulfate, sodium hyaluronate, sodium lactate, sodium palmitate, sodium PCA, sodium polyglutamate, sodium stearate, soluble collagen, sorbic acid, sorbitan laurate, sorbitan oleate, sorbitan palmitate, sorbitan sesquioleate, sorbitan stearate, sorbitol, soybean (glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (helianthus annuus) seed oil, sweet almond (prunus amygdalus dulcis) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (triticum vulgare) germ oil, and ylang ylang (cananga odorata) oil.

### iii. Antioxidants

Non-limiting examples of antioxidants that can be used with the compositions used in the method of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical antioxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### iv. Structuring Agents

In other non-limiting aspects, the compositions used in the method of the present invention can include a structuring agent. Structuring agents, in certain aspects, assist in providing rheological characteristics to the composition to contribute to the composition's stability. In other aspects, structuring agents can also function as an emulsifier or surfactant. Non-limiting examples of structuring agents include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, and mixtures thereof.

### v. Emulsifiers

In some non-limiting aspects, the compositions can include one or more emulsifiers. Emulsifiers can reduce the interfacial tension between phases and improve the formulation and stability of an emulsion. The emulsifiers can be nonionic, cationic, anionic, and zwitterionic emulsifiers (See McCutcheon's (1986); U.S. Pat. Nos. 5,011,681; 4,421,769; 3,755,560). Non-limiting examples include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, PPG-2 methyl glucose ether distearate, ceteth-10, polysorbate 80, cetyl phosphate, potassium cetyl phosphate, diethanolamine cetyl phosphate, polysorbate 60, glyceryl stearate, PEG-100 stearate, and mixtures thereof.

### vi. Silicone Containing Compounds

In non-limiting aspects, silicone containing compounds include any member of a family of polymeric products whose molecular backbone is made up of alternating silicon and oxygen atoms with side groups attached to the silicon atoms. By varying the -Si-O- chain lengths, side groups, and crosslinking, silicones can be synthesized into a wide variety of materials. They can vary in consistency from liquid to gel to solids.

The silicone containing compounds that can be used in the context of the method of the present invention include those described in this specification or those known to a person of ordinary skill in the art. Non-limiting examples include silicone oils (*e.g*., volatile and non-volatile oils), gels, and solids. In preferred aspects, the silicon containing compounds includes a silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (*e.g*., to a particular area such as the skin, hair, or eyes). A "volatile silicone oil" includes a silicone oil have a low heat of vaporization, *i.e.* normally less than about 50 cal per gram of silicone oil. Non-limiting examples of volatile silicone oils include: cyclomethicones such as Dow Corning 344 Fluid, Dow Corning 345 Fluid, Dow Corning 244 Fluid, and Dow Corning 245 Fluid, Volatile Silicon 7207 (Union Carbide Corp., Danbury, Conn.); low viscosity dimethicones, *i.e.* dimethicones having a viscosity of about 50 cst or less (*e.g*., dimethicones such as Dow Corning 200-0.5 cst Fluid). The Dow Corning Fluids are available from Dow Corning Corporation, Midland, Michigan. Cyclomethicone and dimethicone are described in the Third Edition of the CTFA Cosmetic Ingredient Dictionary (incorporated by reference) as cyclic dimethyl polysiloxane compounds and a mixture of fully methylated linear siloxane polymers end-blocked with trimethylsiloxy units, respectively. Other non-limiting volatile silicone oils that can be used in the context of the present invention include those available from General Electric Co., Silicone Products Div., Waterford, N.Y. and SWS Silicones Div. of Stauffer Chemical Co., Adrian, Michigan.

### vii. Essential Oils

Essential oils include oils derived from herbs, flowers, trees, and other plants. Such oils are typically present as tiny droplets between the plant's cells, and can be extracted by several method known to those of skill in the art (*e.g*., steam distilled, enfleurage (*i.e*., extraction by using fat), maceration, solvent extraction, or mechanical pressing). When these types of oils are exposed to air they tend to evaporate (*i.e*., a volatile oil). As a result, many essential oils are colorless, but with age they can oxidize and become darker. Essential oils are insoluble in water and are soluble in alcohol, ether, fixed oils (vegetal), and other organic solvents. Typical physical characteristics found in essential oils include boiling points that vary from about 160° to 240° C and densities ranging from about 0.759 to about 1.096.

Essential oils typically are named by the plant from which the oil is found. For example, rose oil or peppermint oil are derived from rose or peppermint plants, respectively. Non-limiting examples of essential oils that can be used in the context of the present invention include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang ylang. Other essential oils known to those of skill in the art are also contemplated as being useful within the context of the present invention.

### viii. Thickening Agents

Thickening agents, including thickener or gelling agents, include substances that can increase the viscosity of a composition. Thickeners include those that can increase the viscosity of a composition without substantially modifying the efficacy of the active ingredient within the composition. Thickeners can also increase the stability of the compositions of the present invention.

Non-limiting examples of additional thickening agents that can be used in the context of the method of the present invention include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol (see U.S. Pat. Nos. 5,087,445; 4,509,949; 2,798,053; CTFA International Cosmetic Ingredient Dictionary, 4th Ed., 1991). Examples of commercially available carboxylic acid polymers include carbomers, which are homopolymers of acrylic acid crosslinked with allyl ethers of sucrose or pentaerytritol (*e.g*., Carbopol™ 900 series from B. F. Goodrich).

Non-limiting examples of crosslinked polyacrylate polymers include cationic and nonionic polymers. Examples are described in U.S. Pat. Nos. 5,100,660; 4,849,484; 4,835,206; 4,628,078; 4,599,379).

Non-limiting examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids.

Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C₁₀-C₃₀ straight chain or branched chain alkyl group through an ether linkage. Typically these polymers are ethers of C₁₀-C₃₀ straight or branched chain alcohols with hydroxyalkylcelluloses. Other useful polysaccharides include scleroglucans comprising a linear chain of (1-3) linked glucose units with a (1-6) linked glucose every three unit.

Non-limiting examples of gums that can be used with the method of the present invention include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

### b. Pharmaceutical Ingredients

Pharmaceutical ingredients are also contemplated as being useful with the emulsion compositions used in the method of the present invention. Non-limiting examples of pharmaceutical ingredients include anti-acne agents, agents used to treat rosacea, analgesics, anesthetics, anorectals, antihistamines, anti-inflammatory agents including non-steroidal anti-inflammatory drugs, antibiotics, antifungals, antivirals, antimicrobials, anticancer actives, scabicides, pediculicides, antineoplastics, antiperspirants, antipruritics, antipsoriatic agents, antiseborrheic agents, biologically active proteins and peptides, burn treatment agents, cauterizing agents, depigmenting agents, depilatories, diaper rash treatment agents, enzymes, hair growth stimulants, hair growth retardants including DFMO and its salts and analogs, hemostatics, kerotolytics, canker sore treatment agents, cold sore treatment agents, dental and periodontal treatment agents, photosensitizing actives, skin protectant/barrier agents, steroids including hormones and corticosteroids, sunburn treatment agents, sunscreens, transdermal actives, nasal actives, vaginal actives, wart treatment agents, wound treatment agents, wound healing agents, *etc.*

### D. Kits

Kits are also contemplated as being used in certain aspects of the method of the present invention. For instance, a composition of the present invention can be included in a kit. A kit can include a container. Containers can include a bottle, a metal tube, a laminate tube, a plastic tube, a dispenser, a pressurized container, a barrier container, a package, a compartment, a lipstick container, a compact container, cosmetic pans that can hold cosmetic compositions, or other types of containers such as injection or blow-molded plastic containers into which the dispersions or compositions or desired bottles, dispensers, or packages are retained. The kit and/or container can include indicia on its surface. The indicia, for example, can be a word, a phrase, an abbreviation, a picture, or a symbol.

The containers can dispense a pre-determined amount of a composition. In other embodiments, the container can be squeezed (*e.g*., metal, laminate, or plastic tube) to dispense a desired amount of the composition. The composition can be dispensed as a spray, foam, an aerosol, a liquid, a fluid, or a semi-solid. The containers can have spray, pump, or squeeze mechanisms. A kit can also include instructions for using the kit and/or compositions. Instructions can include an explanation of how to apply, use, and maintain the compositions.

### EXAMPLES

### EXAMPLE 1

### (Testing Vehicles and Compositions)

Tables 1 and 2 describe generic skin testing formulations in which a skin active ingredient can be incorporated into to determine the types of skin benefits that can be attributed to the skin active ingredient. These formulations are prepared in such a manner that any resulting skin benefit from topical application of the formula to skin can be directly attributed to the skin active ingredient being tested.

**Table 1***

| **Ingredient** | **% Concentration (by weight)** |
|---|---|
| **Phase A** | |
| Water | q.s. |
| Xanthum gum | 0.1 |
| M-paraben | 0.15 |
| P-paraben | 0.1 |
| Citric acid | 0.1 |

| **Phase B** | |
|---|---|
| Cetyl alcohol | 4.0 |
| Glyceryl stearate + PEG 100 | 4.0 |
| Octyl palmitate | 4.0 |
| Dimethicone | 1.0 |
| Tocopheryl acetate | 0.2 |

| **Phase C**** | |
|---|---|
| Skin Active Ingredients | 2.0 |
| **TOTAL** | **100** |

| | |
|---|---|
| *Procedure for making composition: Sprinkle Xanthum gum in water and mix for 10 min. Subsequently, add all ingredients in phase A and heat to 70-75°C. Add all items in phase B to separate beaker and heat to 70-75°C. Mix phases A and B at 70-75°C. Continue mixing and allow composition to cool to 30°C. Subsequently, add phase C ingredient while mixing. **The skin actives identified throughout this specification can be incorporated into this testing formulation as the skin active ingredient. The actives can be individually used or combined in this testing vehicle. The concentration ranges of the actives can be modified as desired or needed by increasing or decreasing the amount of water. For instance, the combinations that can be used can be: (1) in combination can be *argania spinosa* kernel extract, *cucurbita pepo* seed extract, proline and serine; (2) a second combination can be caffeine, *coffea arabica* seed extract, and *crataegus oxyacantha* fruit extract; (3) a third combination can be *euterpe oleracea* fruit extract, *hydrolyzed myrtus communis* leaf extract, *centella asiatica* extract, *camellia sinensis* leaf extract, and *aesculus hippocastanum* seed extract; (4) a fourth combination tocopheryl acetate and glycerin; and (5) a fifth combination can be *argania spinosa* kernel extract, *cucurbita pepo* seed extract, proline, serine, caffeine, *coffea arabica* seed extract, *crataegus oxyacantha* fruit extract, *euterpe oleracea* fruit extract, *hydrolyzed myrtus communis* leaf extract, *centella asiatica* extract, *camellia sinensis* leaf extract, *aesculus hippocastanum* seed extract, tocopheryl acetate, and/or glycerin or any combination thereof. | |

**Table 2***

| **Ingredient** | **% Concentration (by weight)** |
|---|---|
| **Phase A** | |
| Water | q.s. |
| M -paraben | 0.2 |
| P-paraben | 0.1 |
| Na2 EDTA | 0.1 |
| Shea butter | 4.5 |
| Petrolatum | 4.5 |
| Glycerin | 4.0 |
| Propylene Glycol | 2.0 |
| Finsolve TN | 2.0 |

| **Phase B** | |
|---|---|
| Sepigel 305 | 2.0 |

| **Phase C**** | |
|---|---|
| Skin Active Ingredients | 2.0 |
| **TOTAL** | **100** |

| | |
|---|---|
| * Add ingredients in phase A to beaker and heat to 70-75°C while mixing. Subsequently, add the phase B ingredient with phase A and cool to 30°C with mixing. Subsequently, add phase C ingredient while mixing. **The skin actives identified throughout this specification can be incorporated into this testing formulation as the skin active ingredient. The actives can be individually used or combined in this testing vehicle. The concentration ranges of the actives can be modified as desired or needed by increasing or decreasing the amount of water. For instance, the combinations that can be used can be: (1) in combination can be *argania spinosa* kernel extract, *cucurbita pepo* seed extract, proline and serine; (2) a second combination can be caffeine, *coffea arabica* seed extract, and *crataegus oxyacantha* fruit extract; (3) a third combination can be *euterpe oleracea* fruit extract, *hydrolyzed myrtus communis* leaf extract, *centella asiatica* extract, *camellia sinensis* leaf extract, and *aesculus hippocastanum* seed extract; (4) a fourth combination tocopheryl acetate and glycerin; and (5) a fifth combination can be *argania spinosa* kernel extract, *cucurbita pepo* seed extract, proline, serine, caffeine, *coffea arabica* seed extract, *crataegus oxyacantha* fruit extract, *euterpe oleracea* fruit extract, *hydrolyzed myrtus communis* leaf extract, *centella asiatica* extract, *camellia sinensis* leaf extract, *aesculus hippocastanum* seed extract, tocopheryl acetate, and/or glycerin or any combination thereof. | |

The formulation represented in Table 3 is a non-limiting example of the types of compositions that can be used in the context of the methods of the present invention. Any standard method can be used to prepare such compositions. For instance, simple mixing of the ingredients in a beaker can be used. One should mix such ingredients and add heat as necessary to obtain a homogenous composition.

**Table 3***

| **Ingredient** | **% Concentration (by weight)** |
|---|---|
| Water | 76 |
| C12-15 Alkyl Benzoate | 6 |
| Glycerin | 5.4 |
| Alcohol** | 6 |
| Propylene Glycol | 1.4 |
| Triethanolamine | 1.2 |
| Hydrogenated Lecithin | 1 |
| Caffeine | 1 |
| *Argania spinosa* kernel extract**** | 0.1 |
| *Crataegus oxyacantha* fruit extract**** | 0.1 |
| Tocopheryl acetate | 0.1 |
| *Cucurbita pepo* seed extract**** | 0.055 |
| *Camellia sinensis* leaf extract**** | 0.024 |
| *Coffea Arabica* (coffee) seed extract**** | 0.024 |
| Wintergreen extract | 0.024 |
| *Aesculus hippocastanum* seed extract**** | 0.01 |
| Serine | 0.01 |
| Proline | 0.005 |
| *Centella asiatica* extract**** | 0.005 |
| Hydrolyzed *Myrtus communis* leaf extract**** | 0.002 |
| *Euterpe oleracea* fruit extract**** | 0.001 |
| Additional Ingredients*** | ∼3 |
| **TOTAL** | **100** |

| | |
|---|---|
| *The composition was prepared by using simple mixing procedures. The ingredients were added to a beaker and heated to 70-75°C while mixing. Subsequently, the mixture was cooled to room temperature with constant mixing. Mixing was stopped once a homogenous composition is obtained. **A wide variety of alcohols can be used. In this formulation, the alcohol comprises a combination of stearyl alcohol, cetearyl alcohol, and cetyl alcohol. ***If desired, additional ingredients can be used to form a particular base, formulation, viscosity, *etc.* The additional ingredients can be added at any amounts needed and the water content of the formulation can be adjusted accordingly. In the Table 3 formulation, the following additional base set of ingredients were used in which the total amount of the base set of ingredients were approximately 3 % w/w of the formulation: dimethicone; ceteareth-20; carbomer; diazolidinyl urea; xanthan gum; lauramine oxide; disodium EDTA; fragrance; methylparaben; C9-15 alkyl phosphate; butylene glycol; propylparaben; sodium cocoyl glutamate; and phenoxyethanol. These base set of ingredients were not thought to have contributed to the efficacy results/data provided in Example 2. Further, a person having ordinary skill in the art would be able to use a different set of base set of ingredients to achieve a desired result. ****the following ingredients were used in this formulation and for the assays described in Example 2: (1) for *hydrolyzed myrtus communis* leaf extract, Longevicell C provided by Silab (Cedex, FRANCE); (2) for *argania spinosa* kernel extract, Argatensyl provided by Laboratories Serobiologiques (Paris, FRANCE); (3) for *crataegus oxyacantha* fruit extract, Hawthorne Berry Extract provided by Naturex/Pure World Botanicals (South Hackensack, New Jersey, USA); (4) for *cucurbita pepo* seed extract, Pumpkin Seed Extract provided by Draco Natural Products (San Jose, California, USA); (5) for *euterpe oleracea* fruit extract, Acai Fruit Extract provided by Carrubba Inc. (Milford, Connecticut, USA); and (6) for a combination of camellia sinensis leaf extract, coffea arabica seed extract, centella asiatica extract, and aesculus hippocastanum seed extract, Slimming Phytoamine Biocomplex provided by Alban Muller International (Vincennes, FRANCE). | |

### EXAMPLE 2

### (Efficacy)

The efficacy of the composition described in Table 3 is summarized below in the following Table 4.

**Table 4***

| **Panelist Question** | **Two Weeks of Use** | **Four Weeks of Use** | **Eight Weeks of Use** |
|---|---|---|---|
| Smother Skin | 94% (yes) | DNR** | DNR |
| Toned Skin | DNR | 77% (yes) | DNR |
| Smoother Body Contours*** | DNR | 79% (yes) | DNR |
| Firmer Skin | DNR | 81% (yes) | DNR |
| Firmed Body Contours*** | DNR | DNR | 68% (yes) |
| More Defined Skin | DNR | DNR | 76% (yes) |

| | | | |
|---|---|---|---|
| *Results reported during an independent consumer study, in which 125 women used the Table 4 formulation twice/day for an 8 week period. **DNR is data not requested in survey. ***Equates to reduced appearance of deep lines and wrinkles, pits, and nodules in skin. | | | |

Data also confirms that the Table 3 formulation has the ability to increase the integrity of the dermal-epidermal junction in a person's skin by stimulating the production of proteins and enzymes in dermal and epidermal cells that aid in connecting the dermal layer to the epidermal layer. Testing confirmed that the combination of *argania spinosa* kernel extract, *cucurbita pepo* fruit extract, serine, and proline was a key to this effect. Data also confirms that the Table 3 formulation has the ability to stimulate dermal or epidermal cellular activity in a person's skin.

*In vitro* assays were performed on a selected skin active ingredients. The types of assays and corresponding results are provided in the following paragraphs.

**Antioxidant Capabilities:** An antioxidant assay (Cayman Chemical, #709001) was used to measure the total antioxidant capacity of extracts from *centella asiatica, aesculus hippocastanum,* hydrolyzed *myrtus communis* leaf extract, *camellia sinensis* leaf extract, and *euterpe oleracea* fruit. The assay relies on the ability of antioxidants in the sample to inhibit the oxidation of ABTS® (2,2'-azino-di-[3-ethylbenzthiazoline sulphonate]) to ABTS® + by metmyoglobin. The capacity of the antioxidants in the sample to prevent ABTS® oxidation is compared with that of Trolox, a water-soluble tocopherol analogue, and is quantified as molar Trolox equivalents. Extracts from *centella asiatica, aesculus hippocastanum,* hydrolyzed *myrtus communis* leaf, *camellia sinensis* leaf, and *euterpe oleracea* inhibited oxidation by 26%, 42%, 68%, 70% and 94% respectively.

**Reduction in the Level of Fat Production By Adipose Cells:** Lipolysis is catabolic pathway which involves the breakdown of fat stored in mature adipocytes resulting in the release free fatty acids into the bloodstream. This process plays a central role in the regulation of energy for the subsequent oxidation of the free fatty acids results in a net gain of energy. When human adipocytes are grown *in vitro,* the breakdown of triglycerides leads to release of free fatty acids and glycerol into the medium. This was used as a means to screen the effects of active materials on the lipolysis of fat produced by human adipocytes by measuring the amount of glycerol released into the medium. Extracts from *crataegus oxyacantha* increased lipolysis in cultured human adipocytes by 272.4%.

**Reduction in Production of New Fat Cells:** Adipogenesis is the differentiation process by which pre-adipocyte cells become mature adipocytes. Pre-adipocytes which do not produce lipids can remain as they are or differentiate into mature adipocytes. Only differentiated adipocytes are able to produce fat. Differentiated adipocytes loose their ability to divide. However, they have a very long half-life and the ability to store increasing amounts of lipids. To help prevent cellulite from forming, the maturation of adipocytes can be inhibited. Using this assay, it was discovered that *crataegus oxyacantha* fruit extract inhibits the differentiation of cultured human adipocytes by 60.3% respectively compared to untreated cells.

**Improved Cellular Metabolism and Micro-Circulation:** Energy is required to breakdown fats and produce proteins needed for improved cellular processes. An increase in energy, or cellular metabolism, can occur through multiple cellular pathways. The stimulation of metabolism and improved circulation are both needed to properly stimulate the elimination of wastes and increase absorption of nutrients. Excess fat causes adipose cells to swell which places a strain on the surrounding connective tissue. The resulting sluggish microcirculation allows lymph fluid to leak into the surrounding tissue while hampering the transport of nutrients. Furthermore, inadequate drainage slows the removal of excess fluid and fatty acids released by lipolysis. Caffeine is an effective phosphodiesterase inhibitor which increases cellular levels of cAMP leading to conversion of triglycerides into free fatty acids and glycerol. In addition, *centella asiatica* extract stimulates the body to produce substances that strengthen the collagen fibers and improve circulation of blood.

**Increase Dermal Matrix Proteins Present in Dermal/Epidermal Junction:** The skin contains constricting bands of connective tissue composed of matrix-producing fibroblasts, fat-producing adipocytes and the blood network. Connective tissue can vary in thickness and is held in place by a network of fibers that provides cushion for muscles and organs. This cushion or extracellular matrix is primarily composed of collagen to help provide support and foundation for the skin. When the dermis is strong and structurally sound, fat cells are unable to break through and become visible on the skin surface.

Collagen is the most predominant protein in the connective tissue. It is secreted by fibroblasts where it is matured by other proteins. Extracts of *cucurbita pepo* seed extract increased the production of collagen I by 41.6% secreted by human fibroblasts compared to untreated controls.

Elastin is a connective tissue protein that helps skin resume shape after stretching or contracting. Elastin is also an important load-bearing protein used in places where mechanical energy is required to be stored. Elastin is made by linking many soluble tropoelastin protein molecules, in a reaction catalyzed by lysyl oxidase. *Argania spinosa* kernel extract significantly increased the secreted elastin in cultured human fibroblasts by 285.9%. In addition, it increased activity of the elastin promoter greater than 65%.

Matrix proteins are degraded in the connective tissue by enzymes known as matrix metalloproteinases (MMPs), a group of zinc dependent enzymes (endopeptidases). MMPs are not constitutively expressed in the skin but their activity can be regulated. These enzymes degrade the extracellular matrix in a specific manner; different MMPs degrade specific matrix proteins. Many of the collagen found in the skin (Collagens I, III, IV, and VII) are targets of MMP-3 (Stromelysin 1). Inhibition of the enzyme's activity prevents the destruction of matrix proteins which are essential for the structural foundation of the skin. Extracts of *cucurbita pepo* seeds inhibited >90% of the activity from purified MMP-3 enzyme.

### EXAMPLE 3

### (Additional Assays that Can Be Used To Test Compositions)

The efficacy of the compositions used in the method of the present invention can be determined by methods known to those of ordinary skill in the art. The following are non-limiting assays that can be used in the context of the present invention. It should be recognized that other testing procedures can be used, including, for example, objective and subjective procedures.

**Skin Firmness and Elasticity Assay with a Hargens Ballistometer:** Skin firmness and elasticity can be measured using a Hargens ballistometer, a device that evaluates the firmness and elasticity of the skin by dropping a small body onto the skin and recording its first two rebound peaks. The ballistometry is a small lightweight probe with a relatively blunt tip (4 square mm-contact area) was used. The probe penetrates slightly into the skin and results in measurements that are dependent upon the properties of the outer layers of the skin, including the stratum corneum and outer epidermis and some of the dermal layers.

**Skin Softness/Suppleness Assay with a Gas Bearing Electrodynamometer:** Skin softness/suppleness can be evaluated using the Gas Bearing Electrodynamometer, an instrument that measures the stress/strain properties of the skin. The viscoelastic properties of skin correlate with skin moisturization. Measurements can be obtained on the predetermined site on the cheek area by attaching the probe to the skin surface with double-stick tape. A force of approximately 3.5 gm can be applied parallel to the skin surface and the skin displacement is accurately measured. Skin suppleness can then be calculated and is expressed as DSR (Dynamic Spring Rate in gm/mm).

**Skin Moisture/Hydration Assay**: Skin moisture/hydration benefits can be measured by using impedance measurements with the Nova Dermal Phase Meter. The impedance meter measures changes in skin moisture content. The outer layer of the skin has distinct electrical properties. When skin is dry it conducts electricity very poorly. As it becomes more hydrated increasing conductivity results. Consequently, changes in skin impedance (related to conductivity) can be used to assess changes in skin hydration. The unit can be calibrated according to instrument instructions for each testing day. A notation of temperature and relative humidity can also be made. Subjects can be evaluated as follows: prior to measurement they can equilibrate in a room with defined humidity (*e.g*., 30-50%) and temperature (*e.g*., 68-72°C). Three separate impedance readings can be taken on each side of the face, recorded, and averaged. The T5 setting can be used on the impedance meter which averages the impedance values of every five seconds application to the face. Changes can be reported with statistical variance and significance.

**Skin Dryness, Surface Lines, Skin Smoothness, and Skin Tone Assay:** Skin dryness, surface fine lines, skin smoothness, and skin tone can be evaluated with clinical grading techniques. For example, clinical grading of skin dryness can be determined by a five point standard Kligman Scale: (0) skin is soft and moist; (1) skin appears normal with no visible dryness; (2) skin feels slightly dry to the touch with no visible flaking; (3) skin feels dry, tough, and has a whitish appearance with some scaling; and (4) skin feels very dry, rough, and has a whitish appearance with scaling. Evaluations can be made independently by two clinicians and averaged.

**Skin Smoothness and Wrinkle Reduction Assay With Methods Disclosed in Packman *et al*. (1978):** Skin smoothness and wrinkle reduction can also be assessed visually by using the methods disclosed in Packman and Gams (1978). For example, at each subject visit, the depth, shallowness and the total number of superficial facial lines (SFLs) of each subject can be carefully scored and recorded. A numerical score was obtained by multiplying a number factor times a depth/width/length factor. Scores are obtained for the eye area and mouth area (left and right sides) and added together as the total wrinkle score.

**Appearance of Lines and Wrinkles Assay with Replicas:** The appearance of lines and wrinkles on the skin can be evaluated using replicas, which is the impression of the skin's surface. Silicone rubber like material can be used. The replica can be analyzed by image analysis. Changes in the visibility of lines and wrinkles can be objectively quantified *via* the taking of silicon replicas form the subjects' face and analyzing the replicas image using a computer image analysis system. Replicas can be taken from the eye area and the neck area, and photographed with a digital camera using a low angle incidence lighting. The digital images can be analyzed with an image processing program and are of the replicas covered by wrinkles or fine lines was determined.

**Surface Contour of the Skin Assay with a Profilometer/Stylus Method:** The surface contour of the skin can be measured by using the profilometer/Stylus method. This includes either shining a light or dragging a stylus across the replica surface. The vertical displacement of the stylus can be fed into a computer *via* a distance transducer, and after scanning a fixed length of replica a cross-sectional analysis of skin profile can be generated as a two-dimensional curve. This scan can be repeated any number of times along a fix axis to generate a simulated 3-D picture of the skin. Ten random sections of the replicas using the stylus technique can be obtained and combined to generate average values. The values of interest include Ra which is the arithmetic mean of all roughness (height) values computed by integrating the profile height relative to the mean profile height. Rt which is the maximum vertical distance between the highest peak and lowest trough, and Rz which is the mean peak amplitude minus the mean peak height. Values are given as a calibrated value in mm. Equipment should be standardized prior to each use by scanning metal standards of know values. Ra Value can be computed by the following equation: Rₐ = Standardize roughness; *l*ₘ = the traverse (scan) length; and y = the absolute value of the location of the profile relative to the mean profile height (x-axis).

**Skin Clarity and Reduction in Freckles and Age Spots Assay:** Skin clarity and the reduction in freckles and age spots can be evaluated using a Minolta Chromometer. Changes in skin color can be assessed to determine irritation potential due to product treatment using the a* values of the Minolta Chroma Meter. The a* value measures changes in skin color in the red region. This is used to determine whether a composition is inducing irritation. The measurements can be made on each side of the face and averaged, as left and right facial values. Skin clarity can also be measured using the Minolta Meter. The measurement is a combination of the a*, b, and L values of the Minolta Meter and is related to skin brightness, and correlates well with skin smoothness and hydration. Skin reading is taken as above. In one non-limiting aspect, skin clarity can be described as L/C where C is chroma and is defined as (a²+ b²)^{1/2}

## Claims

1. A method of increasing the integrity of the dermal-epidermal junction in a person's skin by stimulating the production of proteins and enzymes in dermal and epidermal cells that aid in connecting the dermal layer to the epidermal layer comprising topically applying to skin in need thereof a composition comprising:
(a) an effective amount of a combination of plant extracts comprising *argania spinosa* kernel extract and *cucurbita pepo fruit* extract; and
(b) an effective amount of a combination of amino acids comprising serine and proline,
wherein topical application of the composition to skin in need thereof increases the integrity of the dermal-epidermal junction by stimulating the production of proteins and enzymes in dermal and epidermal cells that aid in connecting the dermal layer to the epidermal layer.

2. The method of claim 1, wherein the composition comprises:
0.01% to 1% w/w of *argania spinosa* kernel extract;
0.01% to 1% w/w of *cucurbita pepo fruit* extract; and
0.01% to 1% w/w of a combination of serine and proline.

## Patentansprüche

1. Verfahren zum Verbessern der Integrität des dermalen-epidermalen Übergangs in der Haut einer Person, indem die Produktion von Proteinen und Enzymen in dermalen und epidermalen Zellen stimuliert wird, die beim Verbinden der dermalen Schicht mit der epidermalen Schicht helfen, bei welchem Verfahren auf Haut, die dessen bedarf, eine Zusammensetzung topisch aufgebracht wird, die:
(a) eine wirksame Menge einer Kombination von Pflanzenextrakten, die *Argania-Spinosa*-Kernextrakt und *Cucurbita-Pepo*-Fruchtextrakt umfasst, und
(b) eine wirksame Menge einer Kombination von Aminosäuren, die Serin und Prolin umfassen,
umfasst, wobei die topische Aufbringung der Zusammensetzung auf Haut, die dessen bedarf, die Integrität des dermalen-epidermalen Übergangs verbessert, indem die Produktion von Proteinen und Enzymen in dermalen und epidermalen Zellen stimuliert wird, die beim Verbinden der dermalen Schicht mit der epidermalen Schicht helfen.

2. Verfahren nach Anspruch 1, wobei die Zusammensetzung:
0,01 Gew.-% bis 1 Gew.-% Argania-Spinosa-Kernextrakt,
0,01 Gew.-% bis 1 Gew.-% Cucurbita-Pepo-Fruchtextrakt, und
0,01 Gew.-% bis 1 Gew.-% einer Kombination von Serin und Prolin umfasst.

## Revendications

1. Procédé pour augmenter l'intégrité de la jonction dermo-épidermique dans la peau d'une personne en stimulant la production de protéines et d'enzymes dans les cellules dermiques et épidermiques qui aident à relier la couche dermique à la couche épidermique, comprenant l'application topique, sur la peau en ayant besoin, d'une composition comprenant :
(a) une quantité efficace d'une combinaison d'extraits de plantes comprenant un extrait de grains d'*argania spinosa* et un extrait de fruit de *cucurbita pepo* ; et
(b) une quantité efficace d'une combinaison d'acides aminés comprenant la sérine et la proline,
où l'application topique de la composition sur la peau en ayant besoin augmente l'intégrité de la jonction dermo-épidermique en stimulant la production de protéines et d'enzymes dans les cellules dermiques et épidermiques qui aident à relier la couche dermique à la couche épidermique.

2. Procédé de la revendication 1, dans lequel la composition comprend :
0,01% à 1% p/p d'extrait de grains d'*argania spinosa ;*
0,01% à 1% p/p d'extrait de fruit de *cucurbita pepo ;* et
0,01% à 1% p/p d'une combinaison de sérine et de proline.
